# EUROPEAN PATENT APPLICATION

(11) **EP 3 928 801 A1**
(43) Date of publication of application: **29.12.2021**
(21) Application number: 20197082.9
(22) Date of filing: 19.09.2020
(51) Int. Cl.: A61L 2/025, A61L 2/22

(54) **FOGGING SYSTEM FOR DRY-MIST DISINFECTION**

(30) Priority: 24.06.2020 PL 43443420
(71) Applicant: JFC Polska Sp. z o.o., 05-252 Dabrówka (PL)
(72) Inventor: Majewski, Miros aw, 05-250 Radzymin (PL)
(74) Representative: Domon, Magdalena

(57) **Abstract**

A fogging system for dry mist disinfection comprising a disinfection booth equipped with intake vents (10) located on the side walls (5) and/or the roof (6) of the disinfection booth, and a tank (7) with disinfectant liquid located outside the disinfection booth, where in the tank (7) with disinfectant liquid there is placed a device generating dry mist with a disinfectant, advantageously in the form of an ultrasonic generator, and a device feeding the dry mist into the intake vents, advantageously in the form of a fan; and furthermore, the disinfection booth is equipped with a switch (12) activating the ultrasonic generator and the fan, located inside or outside the disinfection booth.

## Description

The present invention relates to a fogging system for dry-mist disinfection, in particular, for disinfection of people, vehicles, equipment and goods; it is intended for use especially in agriculture, industry and public facilities.

Recurrent outbreaks of both animal and human diseases mean that solutions are needed to prevent the spread of the epidemic and, at the same time, to ensure the smooth functioning of individual sectors of agriculture, industry and public institutions.

In the state of the art, there are known several devices the purpose of which is to provide disinfection of people, devices and equipment.

One of the solutions, used e.g. in agriculture, are disinfectant mats, which are placed in front of the entrance to both farms and farm buildings. As a result, infected biological material is not introduced from outside, and possible pathogenic microorganisms are not spread inside breeding farms. Such mats are used to remove infectious biological material from infected, sick or fallen wild or domestic animals, animal products, soil, mud, manure, etc. from the wheels and wheel arches of machines and means of transport. Such disinfection mats are usually composed of 3 layers, a top one, an absorbent one and a bottom one. The top layer is made of a strong material with hygroscopic properties thus allowing the flow of the disinfection preparation. At the same time, this layer performs the function of a doormat. The absorbent layer is soaked with a disinfectant. The bottom layer is made of waterproof material ensuring that there is no migration of the disinfectant solution outside the mat. Such mats are intended for human use as well as for cars and equipment.

The disadvantage of this type of solution is that it only allows for disinfection of the underside of the shoes or wheels of vehicles whereas it is not possible to disinfect the upper part of machines, goods or, e.g. people's clothes completely.

In order to solve the problem of only partial disinfection, there are in the state of the art booths that enable external disinfection of people as well as vehicles and goods.

Among such solutions there are booths for dry disinfection of the body proposed by the CELIOS Corporation. They consist of a booth equipped with a device for spraying the disinfectant liquid, placed on the side or top wall, and an internal tank for the disinfectant liquid with a capacity of approx. 15 litres.

Another solution is a channel for body disinfection enabling first the measurement of body temperature and then disinfection by using the spray solution described above.

Due to the fact that the disinfectant spraying components are placed on one wall, the disadvantage of this solution is that it is not possible to spray the disinfectant liquid evenly, as a result of which the disinfectant is mainly concentrated on the upper or lateral part of the body.

Another known solution is a device containing an ultrasonic generator which uses ultrasounds to produce microscopic droplets on the surface of the disinfectant, which droplets produce so-called dry fog or dry mist which is then distributed under pressure outside the device with a stream of air produced by a compressor and directed to this generator.

Such devices are used for disinfection of rooms but do not provide the possibility of disinfecting larger objects, in particular machinery/vehicles or goods so other solutions are needed to enable disinfection of larger objects.

The aim of the device according to the invention is to provide such a solution that would enable effective disinfection of people, vehicles, equipment or materials in rooms specially designed for this purpose and located e.g. in the vicinity of farm, industrial and public buildings. At the same time, the aim of the invention is to provide a multifunctional device which it will be possible to use both inside and outside the booth, depending on the need.

Fogging system for dry mist disinfection according to the invention comprising a disinfection booth, a disinfectant tank and an ultrasonic generator, characterised in that the disinfection booth is equipped with intake vents located on the side walls and/or in the roof of the disinfection booth while the disinfectant tank is located outside the disinfection booth where the disinfectant tank is equipped with a device generating dry mist with the disinfectant, advantageously in the form of an ultrasonic generator, and a device feeding dry mist into the intake vents, advantageously in the form of a fan, and furthermore the disinfection booth is equipped with a switch activating the ultrasonic generator and the fan, located inside or outside the disinfection booth.

It is advantageous if the switch activating the ultrasonic generator and the fan are in the form of at least one photocell placed in front of the entrance to the booth. At the request of the customer or when a biocide that is an irritant to the eyes and the respiratory system has to be used, the switch is activated manually.

It is advantageous if the external tank together with the ultrasonic generator and the fan is connected to the disinfection booth through a supply duct.

It is advantageous if the disinfection booth is in the form of a gate, advantageously made from a tank, advantageously a polyethylene one or from laminate material.

It is advantageous if the disinfection booth is in the form of a capsule (with one hole for entering and exiting the booth), advantageously made of polyethylene or a laminate material.

It is even more advantageous if the capsule is made from a polyethylene or laminate tank.

It is even more advantageous if the fogging system for disinfection comprises a disinfection booth permanently or temporarily connected to an external box (cabinet), advantageously made of the same material, advantageously polyethylene.

It is even more advantageous if the disinfection booth is connected to the external box by means of mounting hooks or by means of a suspension and/or is screwed permanently by means of available connecting systems, advantageously by riveting, screwing or welding.

It is advantageous if there is a flexible hose in the outer box, advantageously approx. 2m long, which is connected to the same fogging device so that the disinfectant liquid is delivered outside from the fogging device through the fan and the hose.

It is also advantageous if the disinfection booth is a tunnel made with a frame, side walls and a roof.

It is advantageous if the frame is made in the form of profiles, advantageously aluminium ones, or of stainless or acid resistant steel, or plastic, and the side walls and roof are made of glass or plastic, advantageously tubular polycarbonate, homogeneous polycarbonate, polyethylene, laminate, polycarbonate plates, acrylic glass or a plate.

It is advantageous if the ultrasonic generator producing dry mist with disinfectant and the fan feeding the dry mist to the intake vents are equipped with a time relay.

It is advantageous if the system includes an installation that provides light and/or sound signals when the booth is in use.

It is advantageous if a mat is placed in the base of the disinfection booth to absorb the excess disinfectant and at the same time to disinfect shoes or trolley wheels, advantageously placed in a sump tray for the excess preparation.

It is advantageous if there are platforms placed outside the booth, outside the entrance and/or exit, advantageously in front of and behind the mat.

It is advantageous if there are closures at the front and/or back of the booth, advantageously in the form of PVC strips or a swing door, or a sliding door, or an accordion door.

It is advantageous if the disinfection booth is equipped with lighting which is switched on at the same time when the fan feeding the disinfectant to the intake vents is activated.

It is advantageous if the disinfectant used in the disinfection system contains hypochlorous acid.

The system according to the invention allows for disinfection of people, animals, and objects by means of so-called fogging with dry fog (mist).

The solution according to the invention has many advantages. First of all, placing the tank outside the disinfection booth makes it possible to attach to the booth a tank of any size, adjusted to the individual needs. Such a solution ensures lightness of the structure and allows for easy transport and assembly of the system according to the individual needs. The advantage of this solution is the possibility to carry out the disinfection procedure with a small amount of the disinfectant liquid (biocidal).

The solution according to the invention provides a wide range of possibilities to control the activation of the device that generates and feeds dry fog, e.g. by using a photocell or a manual switch.

The application of a photocell and a time relay enables saving both the disinfectant and electricity, and provides to possibility to adjust the dosing time of the disinfectant. As the photocells have been moved away and lifted from the entrance to the booth, it was possible to eliminate accidental activation of the system due to e.g. moving PVC belts caused by wind force.

Furthermore, the design of the invention, in particular due to the use of a tank with a bottom, gives unlimited possibilities for the location of the tank, e.g. in infectious disease hospitals in the so-called dry zone. There is no need to isolate it as the condensate that may form (e.g. during intensive use of the fogging system and the need to switch it on frequently, i.e. continuous operation of the fog generator) is collected in an appropriately shaped bottom made of a plastic material which acts as a sump tray while disinfecting the bottom of footwear. Moreover, placing platforms at the entrance/exit of the booth, especially in front of and behind the mat that absorbs excess disinfectant, eliminates the risk of tripping and, at the same time, makes it easier to move around with e.g. luggage.

Additionally, by using PVC strips (or plastic curtain with magnetic closure), excessive dispersion of the disinfectant is eliminated thus keeping its concentration inside at the right level.

The use of an external box combined with the body of the booth makes it possible to place in the box accessories for fogging by means of the so-called dry steam in the range of 1 to 15 microns, i.e. a fogging device with mist generators, a tank for the disinfectant, a fan and a control system for the fogging system. Moreover, it is also possible to place a pipe connecting the fogger with the booth where a person is to be disinfected (decontaminated). Such a connection makes it possible to use the booth as a mobile that can be easily transported to different places without the need to disconnect the equipment from the booth.

Thanks to the use of an external box (cabinet) connected to the body of the booth, it is possible to install a system for heating the working medium (heating the disinfectant according to the of the manufacturer's instructions), which makes the device more unique and very well adapted to changing weather conditions, especially if it be located outside a building (for external use).

The solution with the combined external box, which is secured with a lock, means that the device is protected against unauthorised access. Only an authorised and trained person can change the settings or refill the working medium.

In addition, the generator is protected against possible mechanical damage resulting from e.g. accidental kicking or hitting with e.g. a trolley, etc.

The external box also protects the generator and controllers from direct exposure to weather conditions, such as rain.

The application of an additional option of spot disinfection (local fogging) by means of a flexible pipe, advantageously several meters long, allows for disinfection of wheeled vehicles, ambulances, etc., pallets with goods or loading spaces of transport vehicles, which makes the device more functional and unique on the market (disinfection of people and other spaces).

In addition, the unique application of external local fogging by means of a flexible pipe allows for multifunctional use of the disinfection (decontamination) booth.

The use of a combined booth (a body with an external box together with other equipment components, such as a duct supplying the disinfectant in the form of fog, i.e. the so-called dry steam, from the device generating this fog to the inside of the booth, advantageously from the top or from the side) allows for easy transfer of the booth and the possibility of heating the device and the agent, i.e. the disinfectant liquid, at low temperatures.

Advantageously large door of the outside box allow easy access for filling/refilling the agent tank with disinfectant and to the box with power supply and control devices of the fogging/disinfection system.

Due to the design and the possible unlimited capacity of the external tank, and at the same time relatively low consumption of the liquid necessary to produce the disinfectant in the form of fog, the booth can be used in public places with a high flow of people, e.g. at entrances to the underground, railway stations, airports, bus stations, schools, kindergartens, creches, offices, shops, galleries, large industrial plants, agricultural farms, etc.

The solution according to the invention can also be used in stadiums, sports halls, swimming pools, factories, outpatient clinics and hospitals.

The system can also be used in the agricultural market, i.e. as protection against viruses on pig, poultry, cow and horse farms.

The disinfection booth comprised in the system according to the invention can be of any width and length, enabling the disinfection of people, animals, vehicles and equipment. The dimensions of the booth can be adjusted to the individual customer requirements.

The invention in non-limiting embodiments is shown in the drawing in which:

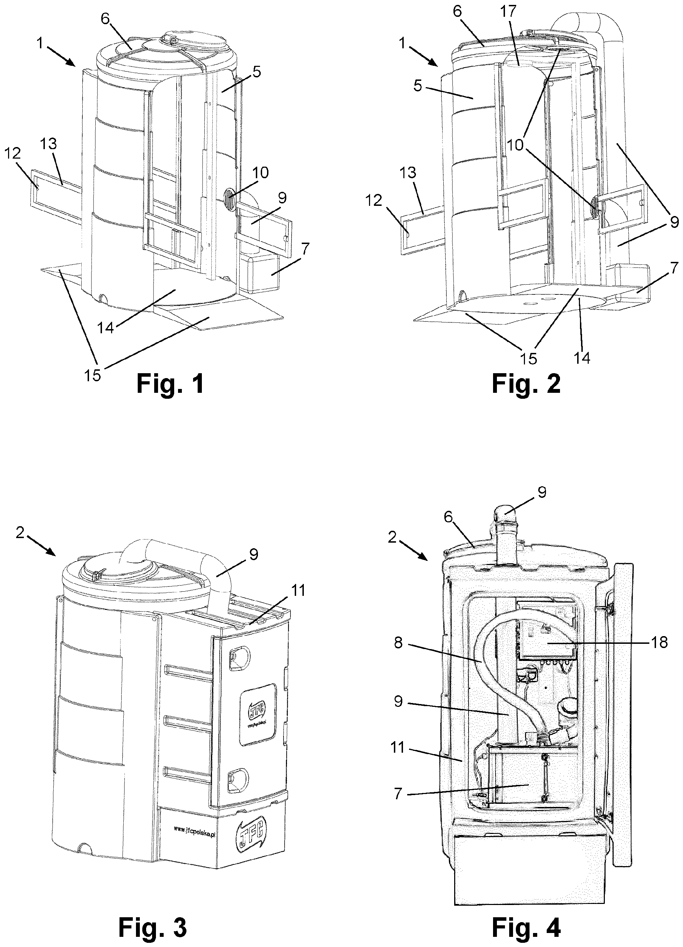

Fig. 1 presents the fogging system with a disinfection booth in the first embodiment in the form of a gate with the external tank shown together with a supply pipe leading dry fog to the intake vent located in the side wall of the booth, with shown handrails incorporating a photocell.
Fig. 2 presents the fogging system with a disinfection booth in the first embodiment in the form of a gate with shown external tank, together with a supply pipe leading the dry fog to the intake vent located both in the side wall of the booth and in the roof of the booth, with shown handrails incorporating a photocell.
Fig. 3 presents the fogging system with a disinfection booth in the second embodiment in the form of a booth containing one entry/exit opening, together with an external box connected thereto.
Fig. 4 presents the fogging system with a disinfection booth in the second embodiment in the form of a booth containing one entry/exit opening, together with an external box combined therewith, in which the external tank is placed together with the supply pipe leading dry fog to the intake vent located both in the side wall of the booth and in the roof of the booth.

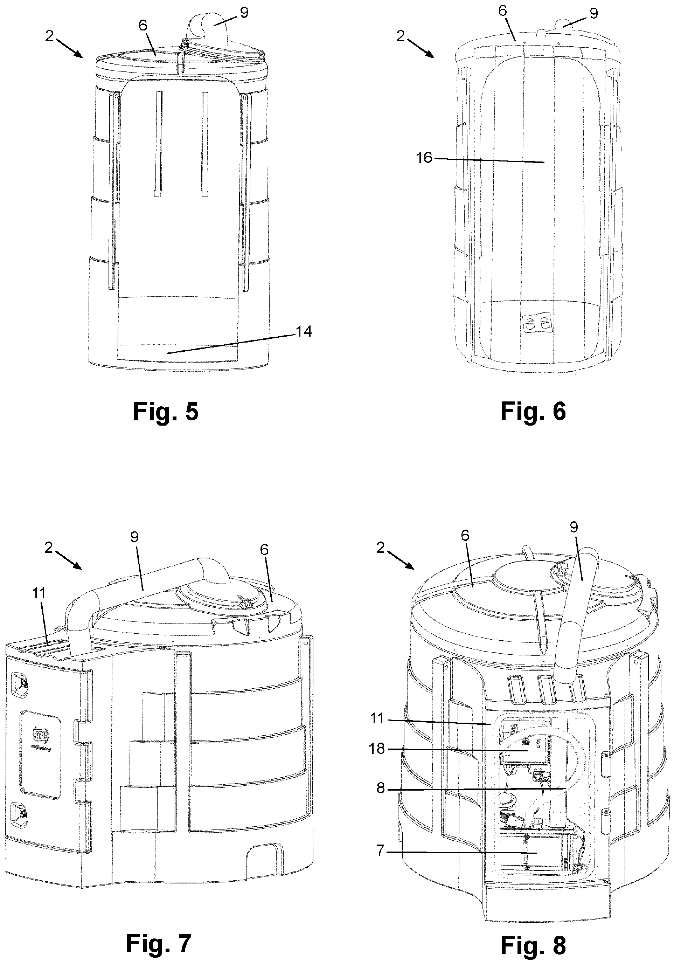

Fig. 5 presents the fogging system with a disinfection booth in the second embodiment in the form of a booth containing one entrance/exit opening, with shown entrance and interior of the booth.
Fig. 6 presents the fogging system with a disinfection booth in the second embodiment in the form of a booth containing one entry/exit opening, with shown entrance covered with a curtain.
Fig. 7 presents the fogging system with a disinfection booth in the third embodiment in the form of a booth comprising one entry/exit opening, together with an external box combined therewith.
Fig. 8 presents the fogging system with a disinfection booth in the third embodiment in the form of a booth containing one entry/exit opening, together with an external box combined therewith, in which the external tank is placed together with the supply pipe leading the dry fog to the intake vent located both in the side wall of the booth and in the roof of the booth.

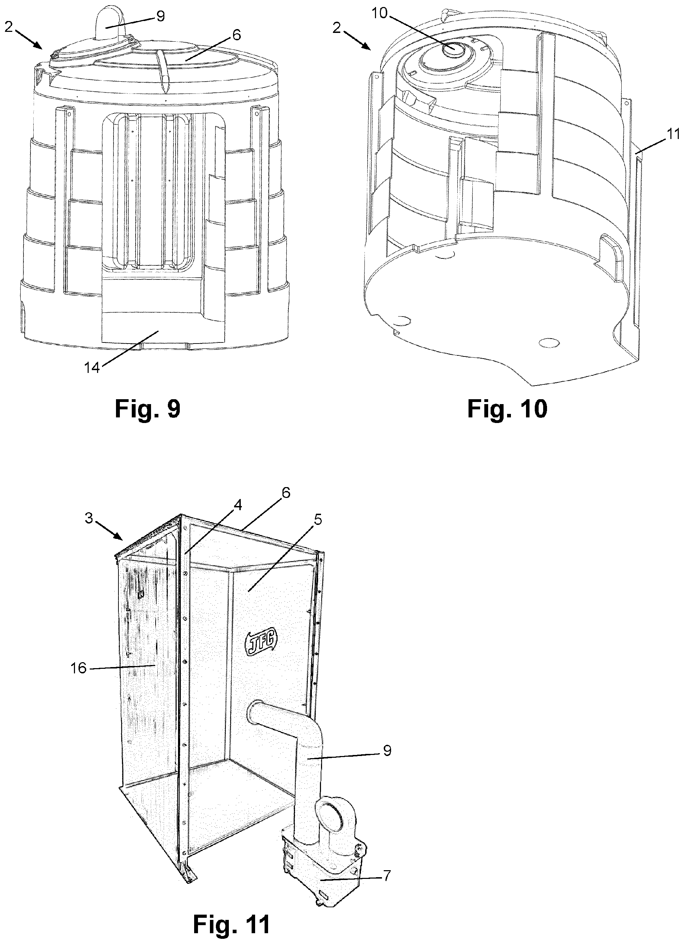

Fig. 9 shows the fogging system with a disinfection booth in the third embodiment in the form of a booth containing one entrance/exit opening, with shown entrance and interior of the booth.
Fig. 10 shows the fogging system with a disinfection booth in the third embodiment in the form of a booth containing one entrance/exit opening, with shown entrance and interior of the booth in the bottom view.
Fig. 11 presents the fogging system with a disinfection booth in the fourth embodiment in the form of a tunnel with shown external device supplying dry fog to the intake vent located in the side wall of the booth, without visible handrails with a photocell.

The fogging system for dry mist disinfection according to the invention is comprised of a disinfection booth and an external tank with disinfectant liquid.

In the first embodiment of the invention, the disinfection booth is in the form of a gate 1 made from a tank, advantageously polyethylene or laminate.

In the second and third embodiments of the invention, the disinfection booth is in the form of a capsule 2 made from a tank, advantageously polyethylene or laminate, whereas the disinfection booth in the second embodiment of the invention differs from the disinfection booth in the third embodiment of the invention by the shape of the tank from which these booths are made.

In the fourth embodiment of the invention, the disinfection booth is in the form of a tunnel 3 made with a frame 4, side walls 5 and a roof 6.

The frame 4 is made in the form of profiles, advantageously aluminium, or stainless or acid resistant steel, or plastic. The side walls 5 and the roof 6 are made of glass or plastic, advantageously tubular polycarbonate, homogeneous polycarbonate, polyethylene, laminate, polycarbonate plates, acrylic glass or a plate.

In all four embodiments of the invention, there is a tank 7 located outside the disinfection booth 1, which tank contains a disinfectant liquid, advantageously containing hypochlorous acid. The tank 7 is equipped with an ultrasonic generator and a fan, not shown in the drawing, located inside the tank 7, which generates and feeds dry mist with a virucidal agent into the booth.

In the non-limiting embodiment of the invention, the disinfectant liquid used in the disinfection system contains advantageously hypochlorous acid. The external tank 7 with the disinfectant liquid is connected to the disinfection booth in the form of a gate 1, capsule 2 or tunnel 3, with a supply duct 9 through intake vents 10 placed in the side wall 5 and/or the roof 6 of the disinfection booth.

In the second and third embodiments of the invention, the external tank 7 with disinfectant liquid is located in an external box 11, combined with the booth in the form of capsule 2, where both the external box 11 and the booth in the form of a capsule 2 are advantageously made of the same material, advantageously polyethylene.

The disinfection booth in the form of a capsule 2 is connected to the outer box 11 by means of mounting hooks, not shown in the drawing, or by means of a suspension and/or is screwed permanently by means of available connecting systems, advantageously by riveting, screwing or welding.

In the second and third embodiments, in the outer box 11 there is a flexible hose 8, advantageously 2 m long (length to be adjusted) which is connected to the tank 6 together with an ultrasonic generator and a fan, generating dry mist with a virucidal agent (or with a disinfection solution). After closing the fogging hole (fogging nozzle) in the booth with a plug, not shown in the drawing, it is possible to disinfect motor vehicles, such as ambulances, loading surfaces and driver's cabs, as well as to carry out local disinfection of pallets with goods, fogging and disinfection of the loading surfaces of delivery vans, passenger cars, etc. by means of the fan through hose 8.

The ultrasonic generator is equipped with a time relay not shown in the drawing.

When a single signal input is delivered, the ultrasonic generator is activated for 1 to 15 seconds with the possibility to extend the operating time by adjusting the time relay.

In the first embodiment, the disinfection booth is equipped with a switch 12, advantageously in the form of two photocells, placed on frame 13 in front of the entrance to the booth, and connected to an ultrasonic generator and a fan, both not shown in the drawing. The photocell can be replaced with a switch activated by a person manually (located in the same place as the photocell or in a place indicated by the user), which may be especially necessary in solutions involving the use of an irritant to the mucous membrane and human respiratory system and requiring eye protection by wearing goggles and a mask to protect the respiratory system.

When the beam emitted by the photocell is blocked, a signal is given to start the ultrasonic generator. The activated ultrasonic generator generates, by a known method, on the surface of the disinfectant liquid located in the external tank 7, so-called dry mist in the form of micro droplets which are then pushed outside by the fan from the external tank 7 into the disinfection booth through the supply ducts 9 and the intake vents 10.

In the disinfection booth, there is placed a mat 14 which absorbs excess preparation, advantageously located in a sump tray, which mat also disinfects shoes or wheels of trolleys.

In front of and behind the mat 14 there are placed platforms 15.

At the front and/or back of the booth there are 16 closures in the form of PVC strips. In other solutions of the system according to the invention, at the front and/or the back of the booth there can be advantageously placed a swing door, or a sliding door, or an accordion door, or curtains - plastic (foil) curtains, e.g. with a magnetic closure.

The disinfection booth is equipped with lighting 17 which is switched on at the same time when the generator and fan that produce and feed the disinfectant to the disinfection booth are activated.

In the second and third embodiments, the system controlling the activation of the ultrasonic generator is located in a switch box 18 located in the 11th external box.

## Claims

1. A fogging system for dry mist disinfection, comprising a disinfection booth, a tank for disinfectant and an ultrasonic generator, **characterised in that** the disinfection booth is equipped with intake vents (10) located on the side walls (5) and/or in the roof (6) of the disinfection booth while the tank (7) containing a disinfectant liquid is located outside the disinfection booth, where in the tank (7) with disinfectant liquid there is placed a device generating dry mist with disinfectant, advantageously in the form of an ultrasonic generator and a dry mist feeding device to the intake vents, advantageously in the form of a fan, and the disinfection booth is further equipped with a switch (12) activating the ultrasonic generator and the fan, located inside or outside the disinfection booth.

2. The fogging system for disinfection according to claim 1, **characterised in that** the switch (12) activating the ultrasonic generator and the fan is in the form of at least one photocell placed in front of the entrance to the booth.

3. The fogging system for disinfection according to claims 1-2, **characterised in that** the external tank (7) together with the ultrasonic generator and the fan is connected to the disinfection booth through a supply duct (9).

4. The fogging system for disinfection according to claims 1-3, **characterised in that** the disinfection booth is in the form of a gate (1), advantageously made from a tank, advantageously polyethylene or laminate.

5. The fogging system for disinfection according to claims 1-3, **characterised in that** the disinfection booth is in the form of a capsule (2) (with one hole for entering and exiting the booth), advantageously made of a tank, advantageously polyethylene or laminate.

6. The fogging system for disinfection according to claims 1-5, **characterised in that** the disinfection booth is permanently or temporarily connected to an outer box (11), advantageously made of the same material, advantageously polyethylene.

7. The fogging system for disinfection according to claim 6, **characterised in that** the disinfection booth is connected to the outer box (11) by means of mounting hooks or by means of a suspension and/or is screwed permanently by means of available connecting systems, advantageously by riveting, screwing or welding.

8. The fogging system for disinfection according to claims 6-7, **characterised in that** in the external box (11) there is a flexible hose (8), advantageously approx. 2m long, connected to the tank (7) with disinfectant liquid and a device generating dry fog so that the disinfectant is delivered outside from the fog generating device, by means of the fan and the hose.

9. The fogging system for disinfection according to claims 1-3, **characterised in that** the disinfection booth is a tunnel (3) comprising a frame (4), side walls (5) and a roof (6).

10. The fogging system for disinfection according to claim 9, **characterised in that** the frame (4) is made in the form of profiles, advantageously aluminium, or stainless or acid resistant steel, or plastic, while the side walls (5) and the roof (6) are made of glass or plastic, advantageously tubular polycarbonate, homogeneous polycarbonate, polyethylene, laminate, polycarbonate plates, acrylic glass or a plate.

11. The fogging system for disinfection according to claims 1-10, **characterised in that** the ultrasonic generator producing dry mist with disinfectant and the fan feeding the dry mist to the intake vents (10) are equipped with a time relay (11).

12. The fogging system for disinfection according to claims 1-11, **characterised in that** it is equipped with an installation providing light and/or sound signal when using the booth.

13. The fogging system for disinfection according to claims 1-12, **characterised in that** in the base of the disinfection booth there is placed a mat (14) which absorbs excess disinfecting preparation and at the same time disinfects the shoes or wheels of the trolleys, advantageously placed in a sump tray for the collected excess preparation.

14. The fogging system for disinfection according to claims 1-13, **characterised in that** outside the booth there are platforms in front of the entrance and/or the exit of the booth (15).

15. The fogging system for disinfection according to claims 1-14, **characterised in that** the closures (16) are located at the front and/or the back of the booth, advantageously in the form of PVC strips, or a swing door, or a sliding door, or an accordion door.

16. The fogging system for disinfection according to claims 1-15, **characterised in that** the disinfection booth is equipped with lighting (17) which is switched on at the same time when the fan feeding the disinfectant to the intake vents (10) is activated.

17. The fogging system for disinfection according to claims 1-16, **characterised in that** the disinfectant used in the disinfection system contains hypochlorous acid.
